# EUROPÄISCHE PATENTANMELDUNG

(11) **EP 0 832 653 A1**
(43) Veröffentlichungstag der Anmeldung: **01.04.1998**
(21) Anmeldenummer: 96250206.8
(22) Anmeldetag: 20.09.1996
(51) Int. Cl.: A61K 38/20, A61K 38/19, A61K 31/00

(54) **Verwenddung von Cytokinen und cytotoxischen Substanzen in einem neuen Verfahren zur Tumorbehandlung**

(71) Anmelder: MAX-DELBRÜCK-CENTRUM FÜR MOLEKULARE MEDIZIN, D-13125 Berlin (DE)
(72) Erfinder: Stein, Ulrike, Dr., 13125 Berlin (DE); Walther, Wolgang, Dr., 13125 Berlin (DE); Schlag, Peter M., Prof. Dr., 13467 Berlin (DE)
(74) Vertreter: Baumbach, Friedrich

(57) **Zusammenfassung**

Die Verwendung einer cytotoxischen, MDR-assoziierten Substanz zur Herstellung eines Therapeutikums für eine Tumortherapie, welche dadurch gekennzeichnet ist, daß a) Tumorzellen eines Tumorpatienten mit einem Cytokin chemosensitiviert werden, b) der Umfang der Expression eines oder mehrerer MDR-assoziierter Gene (z.B. mdr1, Mrp, Lrp) im Tumorgewebe des Patienten in Abhängigkeit vom Beginn der Cytokinbehandlung ermittelt wird, c) der Zeitbereich ermittelt wird, in dem die Expression eines oder mehrer MDR-assoziierter Gene durch die Cytokinbehandlung wesentlich vermindert wird, d) der Patient in dem ermittelten Zeitbereich ("Therapeutisches Fenster") mit einer therapeutisch wirksamen Menge einer cytotoxischen, MDR-assoziierten Substanz, deren Wirksamkeit durch die Expression eines oder mehrerer MDR-assoziierter Gene beeinflußt (verbessert) wird, behandelt wird, führt zu einer verbesserten Tumortherapie.

## Beschreibung

Gegenstand der Erfindung ist ein Verfahren zur Tumorbehandlung mit Cytokinen, Monitoring der Expression Von Arzneimittel-Vielfachresistenz (Multidrug Resistenz, MDR)-assoziierten Genen und nachfolgender Behandlung mit cytotoxischen Substanzen, sowie die Verwendung dieser Substanzen zur Herstellung eines Arzneimittels.

Die Effizienz einer Chemotherapie maligner Erkrankungen ist oft durch die simultane Resistenz gegenüber strukrurell und funktionell nicht verwandten cytotoxischen Verbindungen limitiert. Dieses als Arzneimittel-Vielfachresistenz (Multidrug Resistenz, MDR) bezeichnete Phenomen wird über MDR-assoziierte Gene wie z.B. mdr1, Mrp (MDR-assoziiertes Protein), und/oder Lrp (Lung Resistance Protein) vermittelt. Der klassische Resistenztyp der MDR wird durch die Überexpression des mdr1-Genproduktes, des transmembranen P-Glykoproteins (Pgp), verursacht (U.A. Germann et al., Semin. Cell. Biol. 4 (1993) 63-76). Im Normalgewebe wurde das Pgp vor allem in epithelialen Zellen mit exkretorischen bzw. sekretorischen Funktionen nachgewiesen (A.T. Fojo et al., Proc. Natl. Acad. Sci. USA 84 (1987) 265-269). In Tumoren nahezu aller Lokalisationen, einschließlich Hirntumoren, wurde eine Expression des mdr1-Gens beobachtet (K. Nooter et al., Br. J. Cancer 63 (1991) 663-669 und M. Mousseau et al., Eur. J. Cancer 29A (1993) 753-759). Zur Überwindung der MDR im sogenannten 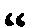 Overcoming 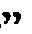 oder 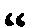 Reversal 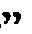 existieren eine Reihe verschiedener Ansätze, die größtenteils auf der Inhibierung der Funktion des Pgp als Drug-Efflux-Pumpe basieren und zum Teil toxische Nebenwirkungen implizieren (J. Kellen Anticancer Res. 13 (1993) 959-961). Zu diesen Resistenz-modifizierenden Agentien zählen beispielsweise Kalzium-Kanal-Blocker, Alkaloide, Pgp-spezifische Antikörper etc.

Von W. Walther und U. Stein wird in Cancer Research and Clinical Oncology (W. Walther et al., Cancer Res. Clin. Oncol. 120 (1994) 471-478) sowie im British Journal of Cancer (U. Stein et al., Br. J. Cancer 74 (1996), in press) der Einfluß von Cytokinen auf die mdr1-Expression in humanen Coloncarcinom-Zellinien beschrieben. Es wurde dort gefunden, daß nach einer Cytokinbehandlung dieser Zellinien nach 48 und 72 Stunden eine merkbare Reduktion der mdr1-Expression zu finden ist. Zu diesen Versuchen wurden die Zellinien LoVo, SW 480, LS174T, HCT15 und HCT116 verwendet. Als Cytokine wurden TNFα, IFNγ und IL-2 eingesetzt. Als cytostatische Substanzen wurden Vincristin und Doxorubicin verwendet. Die Cytokin-vemittelten Effekte auf die mdr1-Genexpression, verbunden mit einer Chemosensitivierung, konnten auch durch den Gentransfer von TNFα in humane Glioblastomzellen U373 MG nachgewiesen werden (W. Walther et al., Int. J. Cancer 61 (1995) 832-839). Es wurde in Übereinstimmung mit anderen Autoren (z.B. C.H. Evans et al., Cancer Res. 52 (1992) 5893-5899 und K.E. Sampson et al., Cancer Lett. 68 (1993) 7-14) darauf hingewiesen, daß die Modulation der mdr1/Pgp-Expression eine interessante Alternative für konventionelle Therapien ist, um die MDR zu überwinden. Ein geeignetes Verfahren hierzu ist weder vorgeschlagen noch nahegelegt. Durch die Erfindung wird ein derartiges Verfahren zur Verfügung gestellt. Damit können Tumoren, die einer derartigen Therapie mit bestimmten MDR-assoziierten cytotoxischen Substanzen in der Chemotherapie bisher nicht oder nur in geringem Maße zugänglich waren, behandelt werden. So wären z.B. colorectale Carcinome, statt bisher, nicht nur einer Therapie mit Antimetaboliten, sondern auch Therapien mit MDR-assoziierten Cytostatika wie z.B. Vincaalkaloiden und/oder Anthrazyklinen besser zugänglich.

Gegenstand der Erfindung ist die Verwendung einer cytotoxischen, MDR-assoziierten Substanz zur Herstellung eines Therapeutikums für eine Tumortherapie, welche dadurch gekennzeichnet ist, daß
a) Tumorzellen eines Tumorpatienten mit einem Cytokin chemosensitiviert werden,
b) der Umfang der Expression eines oder mehrerer MDR-assoziierter Gene (z.B. mdr1, Mrp, Lrp) im Tumorgewebe des Patienten in Abhängigkeit vom Beginn der Cytokinbehandlung ermittelt wird,
c) der Zeitbereich ermittelt wird, in dem die Expression eines oder mehrerer MDR-assoziierter Gene durch die Cytokinbehandlung wesentlich vermindert wird,
d) der Patient in dem ermittelten Zeitbereich ("Therapeutisches Fenster") mit einer therapeutisch wirksamen Menge einer cytotoxischen, MDR-assoziierten Substanz, deren Wirksamkeit durch die Expression eines oder mehrerer MDR-assoziierter Gene beeinflußt (verbessert) wird, behandelt wird.

Ein weiterer Gegenstand der Erfindung ist ein Verfahren zur Tumortherapie bei Patienten, mit einer MDR-assoziierten cytotoxischen Substanz, dadurch gekennzeichnet, daß
a) Tumorzellen eines Tumorpatienten mit einem Cytokin chemosensitiviert werden,
b) der Umfang der Expression eines oder mehrerer MDR-assoziierter Gene (z.B. mdr1, Mrp, Lrp) im Tumorgewebe des Patienten in Abhängigkeit vom Beginn der Cytokinbehandlung ermittelt wird,
c) der Zeitbereich ermittelt wird, in dem die Expression eines oder mehrer MDR-assoziierter Gene durch die Cytokinbehandlung wesentlich vermindert wird,
d) der Patient in dem ermittelten Zeitbereich ("Therapeutisches Fenster") mit einer therapeutisch wirksamen Menge einer cytotoxischen, MDR-assoziierten Substanz, deren Wirksamkeit durch die Expression eines oder mehrerer MDR-assoziierter Gene beeinflußt (verbessert) wird, behandelt wird.

Es wurde überraschenderweise gefunden, daß nach Behandlung eines Tumorpatienten ein Patienten-charakteristischer, zeitlicher Verlauf der Expression eines oder mehrerer MDR-assoziierter Gene in seinen Tumorzellen beobachtet wird. Es zeigt sich, daß nach Cytokinbehandlung ein 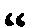 Therapeutisches Fenster 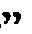 bestimmt werden kann, welches Patienten-spezifisch ist und für nachfolgende Cytostatikabehandlungen vorteilhaft ausgenutzt werden kann. Dies ist insbesondere dann von großer Bedeutung, wenn das Tumorgewebe des Patienten entfernt wird und eine Cytostatikabehandlung zur Vermeidung einer Progression erfolgen soll. Es hat sich überraschenderweise gezeigt, daß auch in diesen Fällen auf das bereits vor der Entfernung des Tumors ermittelte 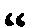 Therapeutische Fenster 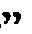 bei diesen nachfolgenden Behandlungen mit einer oder mehreren MDR-assoziierten, cytotoxischen Substanzen Bezug genommen werden kann.

Es hat sich gezeigt, daß mit dem erfindungsgemäßen Verfahren insbesondere gastrointestinale Tumoren, Melanome, Sarkome und Mammacarcinome behandelt werden können.

Unter einer cytotoxischen Substanz ist eine Substanz zu verstehen, welche als Medikament zur Therapie maligner Erkrankungen eingesetzt wird und deren Wirkung aufder Zytotoxizität vor allem gegenüber Tumorzellen beruht. Derartige cytotoxische Substanzen, die in das Spektrum der Multidrug Resistenz gehören, sind beispielsweise Daunorubicin, Doxorubicin, Mitoxantron, Etoposid, Tenoposid, Vinblastin, Vincristin, Actinomycin D, Mitomycin C, Taxol (Paclitaxel), Topotecan, Colchicin, Emetin, Ethidiumbromid, Puromycin, Gramicidin D, Valinomycin und Mithramycin. Derartige Substanzen und ihre Wirkung sind beispielsweise von U.A. Germann in European Journal of Cancer 32A (1996) 927-944 beschrieben. Ebenfalls geeignete cytotoxische Substanzen können Cis-Platin-Verbindungen sein.

Unter einem Cytokin ist eine Substanz zu verstehen, welche als zellulärer, körpereigener Botenstoff immunstimulierende oder auch cytostatische Eigenschaften besitzt und für die cytotoxische bzw. cytostatische Tumortherapie eingesetzt werden kann. Geeignete Cytokine sind beispielsweise Interleukine wie IL-2, Interferone wie IFNα und IFNγ, Tumornekrosefaktoren wie TNFα etc.

Unter einem Multidrug Resistenz-assoziierten Gen (MDR-assoziierten Gen) ist ein Gen zu verstehen, welches eine Protein kodiert, das kausal an der Entwicklung des Multidrug Resistenz-Phenotyps beteilgt ist; wie z.B. als Energie-abhängige transmembrane Drug-Efflux-Pumpe (mdr1, U. A. Germann Eur. J. Cancer 32A (1996) 927-944; Mrp, D. W. Loe et al., Eur. J. Cancer 32A (1996) 945-957) oder das den nukleo-cytoplasmatischen Transport der Droge bewirkt (z.B. Lrp, M. A. Izquierdo et al., Eur. J. Cancer 32A (1996) 979-984).

Die Expression von MDR-assoziierten Genen kann auf beiden Expressionsebenen (mRNA und Protein) mit bekannten molekularbiologischen Techniken ermittelt werden, z.B. mit RT-PCR und/oder Northern Hybridisierung mit isolierter RNA bzw. mRNA, und/oder mit in situ RT-PCR an Kryoschnitten der Tumorproben. Auf der Proteinebene kann der Nachweis der MDR-assoziierten Genprodukte z.B. mit der Technik des Western Blotting mit isolierten Proteinen und/oder mit der Methode der Immunhistochemie an Kryoschnitten, jeweils unter Zuhilfenahme der entsprechenden spezifischen Antikörper, erfolgen.

Der Zeitbereich, in dem die Expression der MDR-assoziierten Gene wesentlich vermindert ist, kann ermittelt werden durch eine zeitabhängige in vitro Analyse von Tumorproben, die z.B. als Stanzbiopsien täglich (vor der Behandlung und 24, 48 und 72 Stunden nach der Cytokin-Applikation) gewonnen werden; es kommen die oben genannten Methoden zum Einsatz.

Unter wesentlich vermindert ist zu verstehen, wenn eine mindestens zweifache Reduktion der mRNA und/oder der Proteine der entsprechenden MDR-assoziierten Gene nachgewiesen wird.

Die Ermittlung der Expression der MDR-assoziierten Gene erfolgt im Tumorgewebe des Patienten. Hierzu wird zweckmäßig eine Biopsie durchgeführt. An Kryoschnitten kann die mRNA der MDR-assoziierten Gene mittels der in situ RT-PCR und die entsprechenden Genprodukte mittels der Immunhistochemie nachgewiesen werden. Neben diesen Aussagen zur Heterogenität des Tumorzellareale und der Expression dieser Gene im Tumorzellverband wird mit quantitativen Methoden (z.B. RT-PCR mit isolierter RNA) die Expressionshöhe der Gene unter Zuhilfenahme externer und interner Standards bestimmt.

Die Cytokinbehandlung kann erfolgen mit einem einzelnen Cytokin, vorzugsweise IL-2, IFNα, IFNγ oder TNFα, oder einer Kombination davon, vorzugsweise TNFα/IFNγ oder IFNα/IL-2.

Die Behandlung kann systemisch oder lokal (z.B. Perfusion mit Cytokinen oder Cytokingentherapie) erfolgen.

Es ist bevorzugt, im Schritt a) Tumorgewebe des Patienten systemisch oder lokal mit einem Cytokin oder Kombinationen davon zu behandeln und im Schritt d) die cytotoxische Substanz oder Kombinationen davon lokal oder systemisch zu verwenden.

Das Cytokin wird in einer therapeutisch wirksamen Konzentration eingesetzt, wodurch die Tumorzellen chemosensitiviert werden.

Die lokale Applikation des Cytokins erfolgt zweckmäßig intratumoral, wobei entweder das Cytokin selbst oder eine Nukleinsäure, welche die Expression des gewünschten Cytokingens in der Tumorzelle vermittelt, verwendet wird (z.B. lokale Gentherapie, gene gun).

Die cytotoxische Substanz wird in einer therapeutisch wirksamen Konzentration, durch welche die Tumorzellen in vivo in beträchtlichem Umfang zerstört werden können, eingesetzt.

Das folgende Beispiel und die genannten Publikationen erläutern die Erfindung, deren Schutzumfang sich aus den Patentansprüchen ergibt, weiter. Das beschriebene Verfahren ist als Beispiel zu verstehen, das auch noch nach Modifikation den Gegenstand der Erfindung beschreibt.

### Beispiel 1

### Individuelle, Patienten-spezifische Zeitverläufe der mdr1-Genexpression nach Cytokin (IL-2)-Applikation am Beispiel des malignen Melanoms

| Patient | Untersuchung | | mdr1-Expression | | |
|---|---|---|---|---|---|
| | | vor IL-2 Behandlung | nach 1. Tag | nach 2. Tag | nach 3. Tag |
| 1 | 1. | ++ | ++ | + | ++ |
| 1 | 2. | ++ | n.d. | n.d. | n.d. |
| 2 | 1. | +++ | + | ++++ | ++++ |
| 2 | 2. | +++ | + | ++ | ++++ |
| 2 | 3. | +++ | + | ++ | n.d. |
| 3 | 1. | ++ | ++++ | ++++ | ++++ |
| 4 | 1. | - | | | |
| 5 | 1. | ++++ | ++ | ++ | - |
| 5 | 2. | ++++ | ++ | + | n.d. |
| - = negativ, keine mdr1-Genexpression nachweisbar + = mdr1-Genexpression nachweisbar, Expressionshöhen (+, ++, +++, ++++) werden in Bezug auf die mitgeführten Standards (Gene mit unbeeinflußter Expression wie β-Aktin und β-Mikroglobulin, Standardabweichung 20%) ermittelt. n.d. = kein Tumormaterial verfügbar (not determined) | | | | | |

Der Einsatz der Cytokinmengen in der Tumortherapie ist sehr variabel, hängt z.B. von der Applikationsart ab (i.v., s.c., bolus etc.) und wird oft individuell für den Patienten festgelegt (M.H. Oppenheim et al., Oncology 51 (1994) 154-169; S. Sone et al., Oncology 51 (1994) 170-176; U. Hieber et al., Oncology 51 (1994) 142-153; S.S. Agarwala et al., Oncology 51 (1994) 129-136).

Die Bereiche für die einzelnen Cytokine sind für:

| | | | |
|---|---|---|---|
| IL-2 | von | 3-20 x 10⁶ IU/m²/Tag | systemisch |
| | | 1000 - 30.000 IU | lokal |
| TNFα | von | 5 - 250 µg/m² | systemisch |
| | | 100 - 250 µg/m² | lokal |
| Interferone | von | 2 - 50 x 10⁶ IU/m²/Tag | systemisch |
| | | 6 - 10 x 10⁶ IU | lokal |

### Referenzliste

S.S. Agarwala et al., Oncology 51 (1994) 129-136
C.H. Evans et al., Cancer Res. 52 (1992) 5893-5899
A.T. Fojo et al., Proc. Natl. Acad. Sci. USA 84 (1987) 265-269
U.A. Germann et al., Semin. Cell. Biol. 4 (1993) 63-76
U.A. Germann Eur. J. Cancer 32A (1996) 927-944
U. Hieber et al., Oncology 51 (1994) 142-153
M. A. Izquierdo et al., Eur. J. Cancer 32A (1996) 979-984
J. Kellen Anticancer Res. 13 (1993) 959-961
D. W. Loe et al., Eur. J. Cancer 32A (1996) 945-957
M. Mousseau et al., Eur. J. Cancer 29A (1993) 753-759
K. Nooter et al., Br. J. Cancer 63 (1991) 663-669
M.H. Oppenheim et al., Oncology 51 (1994) 154-169
K.E. Sampson et al., Cancer Lett. 68 (1993) 7-14
S. Sone et al., Oncology 51 (1994) 170-176
U. Stein et al., Br. J. Cancer 74 (1996), in press
U. Stein et al., J. Natl. Cancer Inst 88 (1996), in press
W. Walther et al., Cancer Res. Clin. Oncol. 120 (1994) 471-478
W. Walther et al., Int. J. Cancer 61 (1995) 832-839

## Patentansprüche

1. Verwendung einer cytotoxischen, MDR-assoziierten Substanz zur Herstellung eines Therapeutikums für eine Tumortherapie, welche dadurch gekennzeichnet ist, daß
a) Tumorzellen eines Tumorpatienten mit einem Cytokin chemosensitiviert werden,
b) der Umfang der Expression eines oder mehrerer MDR-assoziierter Gene (z.B. mdr1, Mrp, Lrp) im Tumorgewebe des Patienten in Abhängigkeit vom Beginn der Cytokinbehandlung ermittelt wird,
c) der Zeitbereich ermittelt wird, in dem die Expression eines oder mehrerer MDR-assoziierter Gene durch die Cytokinbehandlung wesentlich vermindert wird,
d) der Patient in dem ermittelten Zeitbereich ("Therapeutisches Fenster") mit einer therapeutisch wirksamen Menge einer cytotoxischen, MDR-assoziierten Substanz, deren Wirksamkeit durch die Expression eines oder mehrerer MDR-assoziierter Gene beeinflußt (verbessert) wird, behandelt wird.

2. Verwendung nach Anspruch 1, dadurch gekennzeichnet, daß die cytotoxische Substanz ein Anthracyclin, ein Epipodophyllotoxin, ein Vincaalkaloid oder eine Cis-Platin-Verbindung ist und als Cytokin IL-2, GM-CSF, TNFα, IFNα oder IFNγ bzw. eine Kombination dieser Stoffe eingesetzt wird.

3. Verwendung nach den Ansprüchen 1 oder 2, dadurch gekennzeichnet, daß im Schritt a) das Tumorgewebe des Patienten lokal oder systemisch und im Schritt d) lokal oder systemisch behandelt wird.

4. Verwendung nach den Ansprüchen 1-3, dadurch gekennzeichnet, daß die Schritte a) - d) ggf. mehrfach wiederholt werden.

5. Verwendung nach den Ansprüchen 1-4, dadurch gekennzeichnet, daß in Schritt c) der Zeitbereich ermittelt wird, in dem die Expression von einem/mehrerem MDR-assoziierten Gene wesentlich vermindert ist und in Schritt d) im genannten Bereich eine oder eine Kombination von MDR-assoziierten cytotoxischen Substanzen gegeben wird, deren Wirkung durch die in Schritt c) genannten MDR-assoziierten Gene wesentlich beeinflußt werden.

6. Verfahren zur Tumortherapie bei einem Patienten mit einer MDR-assoziierten cytotoxischen Substanz, dadurch gekennzeichnet, daß
a) Tumorzellen eines Tumorpatienten mit einem Cytokin chemosensitiviert werden,
b) der Umfang der Expression eines oder mehrerer MDR-assoziierter Gene im Tumorgewebe des Patienten durch die Cytokinbehandlung in vitro ermittelt wird,
c) der Zeitbereich ermittelt wird, in dem die Expression eines oder mehrerer MDR-assoziierter Gene wesentlich vermindert ist,
d) der Patient in dem ermittelten Zeitbereich mit einer therapeutisch wirksamen Menge einer cytotoxischen, MDR-assoziierten Substanz, deren Wirksamkeit durch die verminderte Expression eines oder mehrerer MDR-assoziierter Gene beeinflußt wird, behandelt wird.

7. Verfahren nach Anspruch 6, dadurch gekennzeichnet, daß die cytotoxische Substanz ein Anthracyclin, ein Epipodophyllotoxin, ein Vincaalkaloid oder eine Cis-Platin-Verbindung ist und als Cytokin IL-2, GM-CSF, IFNα, IFNγ oder TNFα bzw. eine Kombination dieser Stoffe eingesetzt wird.

8. Verfahren nach den Ansprüchen 6-7, dadurch gekennzeichnet, daß im Schritt a) und/oder d) das Tumorgewebe des Patienten lokal oder systemisch behandelt wird.

9. Verfahren nach den Ansprüchen 6-8, dadurch gekennzeichnet, daß nach Schritt c) das genannte Tumorgewebe des Patienten weitgehend oder vollständig entfernt werden kann.

10. Verfahren nach den Ansprüchen 6-9, dadurch gekennzeichnet, daß in Schritt c) der Zeitbereich ermittelt wird, in dem die Expression von mehrerem MDR-assoziierten Genen wesentlich vermindert ist, und in Schritt d) im genannten Bereich eine oder mehrere MDR-assoziierte cytotoxische Substanzen gegeben werden, deren Wirkung durch die in Schritt c) genannten MDR-assoziierten Gene wesentlich beeinflußt wird.
